# EUROPÄISCHE PATENTSCHRIFT

(11) **EP 0 792 882 B1**
(45) Veröffentlichungstag und Bekanntmachung des Hinweises auf die Patenterteilung: **25.04.2001**
(21) Anmeldenummer: 97250040.9
(22) Anmeldetag: 21.02.1997
(51) Int. Cl.: C07D 493/08, A61K 6/083

(54) **Polymerisierbare Hybridmonomere**
Polymerisable hybridmonomers
Monomères hybrides polymerisables

(30) Priorität: 22.02.1996 DE 19608313
(43) Veröffentlichungstag der Anmeldung: 03.09.1997
(73) Patentinhaber: Ivoclar Vivadent AG, 9494 Schaan (LI)
(72) Erfinder: Moszner, Norbert, Prof. Dr., 9492 Eschen (LI); Rheinberger, Volker, Dr., 9490 Vaduz (LI); Zeuner, Frank, Dr., 9490 Vaduz (LI)
(74) Vertreter: UEXKÜLL & STOLBERG

(56) Entgegenhaltungen:
- US-A- 3 681 438
- US-A- 4 054 233
- US-A- 4 808 638

## Beschreibung

Die Erfindung betrifft polymerisierbare Hybridmonomere, ein Verfahren zu deren Herstellung, deren Verwendung insbesondere als Dentalmaterial, ein diese enthaltendes Dentalmaterial sowie aus diesen erhältliche Polymere oder Copolymere.

Verbindungen mit mindestens zwei nach gleichem Mechanismus polymerisierenden Gruppen, wie Divinyl-Verbindungen, Di-, Tri- oder Tetra(meth)acrylate, Bismaleinimide oder Diepoxide, finden als Vernetzermonomere u.a. bei der Herstellung von Klebstoffen, Lacken und Kompositen verbreitet Verwendung (vgl. S.C. Ling, E.M. Pearce, "High-Performance Thermosets, Chemistry, Properties, Applications" in S.P. Pappas (Editor), Radiation Curing - Science and Technology, Plenum Press, New York-London 1992). Demgegenüber sind Monomere mit mindestens zwei nach unterschiedlichen Mechanismen polymerisierenden Gruppen, sogenannte ambifunktionelle Monomere oder Hybridmonomere, für die gezielte Synthese von vernetzbaren Polymeren von Interesse.

In Abhängigkeit von der Art der polymerisierbaren Gruppen kann die ein- oder zweistufige Synthese von Polymernetzwerken aus Hybridmonomeren durch Kombination beispielsweise von radikalischer und kationischer Vinylpolymerisation, z.B. bei Vinyloxyethylmethacrylat, von radikalischer Vinylpolymerisation und kationischer Ringöffnungspolymerisation, z.B. bei Glycidylmethacrylat, von anionischer Gruppentransferpolymerisation und radikalischer Polymerisation, z.B. bei Acryloyloxyethylmethacrylat oder von ringöffnender Metathesepolymerisation und radikalischer Ringöffnungspolymerisation, z.B. bei Cyclooct-5-enylmethacrylat, erreicht werden.

In diesem Zusammenhang sind auch bicyclische Verbindungen mit Bicyclo[2.2.1]hept-2-enyl- (Norbornenyl) oder 7-Oxa-bicyclo[2.2.1]hept-2-enyl-Gruppen sowie solche mit Bicyclo[2.2.1]hept-2,5-dienyl-Gruppen (Norbornadienyl) von Interesse, da sie als Monomere für die ringöffnende Metathesepolymerisation geeignet sind (D.J. Brunelle (Ed.), Ring-Opening Polymerization, Hanser Pub. Munich etc. 1993, S. 129). Darüber hinaus können Norbornen-Verbindungen gemäß US-A-4 808 638 auch als reaktive En-Komponenten für die schrumpfungsarme Thiol-En-Polymerisation verwendet werden.

Hybridmonomere mit drei nach unterschiedlichen Mechanismen polymerisierbaren Gruppen sind allerdings bisher nicht bekannt geworden.

Der Erfindung liegt die Aufgabe zugrunde, polymerisierbare Hybridmonomere bereitzustellen, die bei Raumtemperatur härtbar sind, mittels radikalischer Polymerisation in Kombination mit entweder ionischer Polymerisation oder ringöffnender Metathesepolymerisation polymerisiert und als Bestandteil von Dentalmaterialien, insbesondere als haftungserhöhende Komponente von Dentinadhäsiven, eingesetzt werden können.

Diese Aufgabe wird durch die polymerisierbaren Hybridmonomere nach den Ansprüchen 1 bis 3 gelöst.

Gegenstand der vorliegenden Erfindung sind ebenfalls das Verfahren zur Herstellung der polymerisierbaren Hybridmonomere nach Anspruch 4, deren Verwendung nach den Ansprüchen 5 und 6, Dentalmaterialien mit Gehalt an diesen nach Anspruch 7 und Polymere oder Copolymere nach Anspruch 8, welche durch Polymerisation oder Copolymerisation der Hybridmonomere erhältlich sind.

Die erfindungsgemäßen polymerisierbaren Hybridmonomere sind Verbindungen der folgenden Formel (I), dazu stereoisomere Verbindungen und beliebige Mischungen von allen diesen 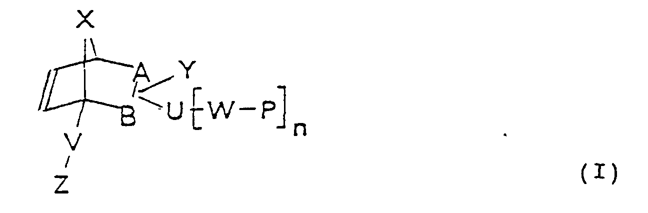 wobei A-B, X, Z, V, Y, R, U, R¹, W, P und n unabhängig voneinander die folgenden Bedeutungen haben:
- A-B =: C-C oder C=C;
- X =: CH₂ oder O;
- Z =: CH₂=CH-CO- oder CH₂=C(CH₃)-CO-;
- V =: CH₂-O oder CH₂-NH;
- Y =: H, substituiertes oder unsubstituiertes C₁- bis C₁₂-Alkyl, substituiertes oder unsubstituiertes C₆-bis C₁₄-Aryl, Halogen, NO₂, NH₂, NR₂, OH, OR, CN, CHO, CO-R, COOH, CO-NH₂, CO-OR, CH₂=CH-, CH₂=CH-CO-, CH₂=C(CH₃)-CO-, SH oder S-R,
wobei
R = substituiertes oder unsubstituiertes C₁-bis C₁₂-Alkyl oder substituiertes oder unsubstituiertes C₆- bis C₁₄-Aryl;
- U =: CO-R¹, CO-NHR¹, CO-OR¹, O-CO-NHR¹, NH-CO-OR¹, O-R¹, S-R¹ oder entfällt,
wobei
R¹ = C₁- bis C₅-Alkylen oder -Oxyalkylen oder C₆- bis C₁₂-Arylen;
- W =: O, NH, CO-O, CO-NH, O-CO-NH oder entfällt;
- P =: eine (Meth)acryl-, Vinyl-, Allyl-, Allylether-, Vinylether, Epoxy- oder Styryl-Gruppe; und
- n =: 1 bis 4, wobei die bei den Resten Y und R gegebenenfalls vorhandenen Substituenten COOH, OH, Halogen, C₁- bis C₁₂-Alkoxy, -N⁺-(C₁- bis C₁₂-Alkyl)₃,
-O-P=O(OH)₂ und/oder -P=O(OH)₂ sind. Es ist möglich, daß Y und R mehrfach substituiert sind.

Dabei decken die obigen Formeln nur solche Verbindungen ab, die mit der Valenzlehre zu vereinbaren sind. Weiter steht Formel (I) für die beiden Stellungsisomere 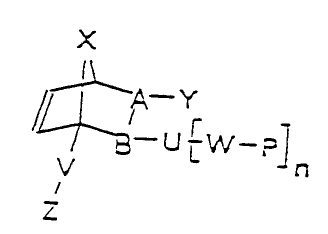 und 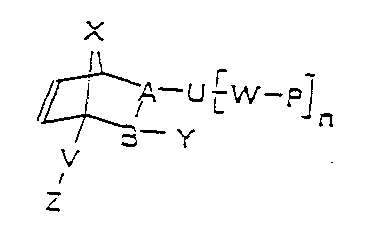 Das gilt entsprechend auch für die weiteren in der Beschreibung und den Ansprüchen angegebenen Formeln, bei denen die in Formel (I) verwendete Form der Darstellung zur Erfassung der Stellungsisomere benutzt wird. Ferner sind die Gruppen Y und U unabhängig voneinander in endo- oder exo-Stellung gebunden.

Das Strukturelement [W-P]ₙ bedeutet, daß U n-fach mit W-P substituiert ist. Für den Fall, daß U= entfällt, ist entweder das Kohlenstoffatom A oder B n-fach mit W-P substituiert.

Typischerweise liegen die erfindungsgemäßen Hybridmonomere in Form von Stereoisomerengemischen, insbesondere als Racemate, vor.

Für die oben angegeben Variablen der Formel (I) existieren unabhängig voneinander wählbare bevorzugte Definitionen, und diese sind wie folgt:
- A-B =: C-C oder C=C,
- X =: O,
- Z =: CH₂=C(CH₃)-CO-,
- V =: CH₂-O,
- Y =: COOH, CN oder CO-NH₂,
- R =: substituiertes oder unsubstituiertes C₁- bis C₄-Alkyl, U = CO-OR¹ oder CO-NHR¹,
- R¹ =: C₁- bis C₃-Alkylen,
- W =: O, NH, CO-O oder entfällt,
- P =: Vinylether-, Epoxy-, Allyl-, Styryl- oder (Meth)acryl-Gruppe; und/oder
- n =: 1 oder 2.

Bevorzugte Verbindungen sind demgemäß solche, bei denen mindestens eine der Variablen der Formel (I) die vorstehend beschriebene bevorzugte Definition aufweist.

Besonders bevorzugte erfindungsgemäße Monomere sind solche, die mindestens drei nach unterschiedlichen Mechanismen polymerisierbare Gruppen enthalten.

Die erfindungsgemäßen Hybridmonomere (I) werden hergestellt, indem man zunächst durch Diels-Alder-Reaktion der substituierten Dien(meth)acryl-Verbindung (II) mit dem substituierten Dienophil (III) eine entsprechend substituierte Norbornen- oder Norbornadien-Verbindung (IV) als Zwischenstufe herstellt, die dann im Rahmen einer normalen nucleophilen Substitution (vgl. Autorenkollektiv, Organikum, Deutscher Verlag der Wissenschaften, Berlin 1973) mit dem polymerisationsgruppenhaltigen Edukt P-W-H umgesetzt wird.

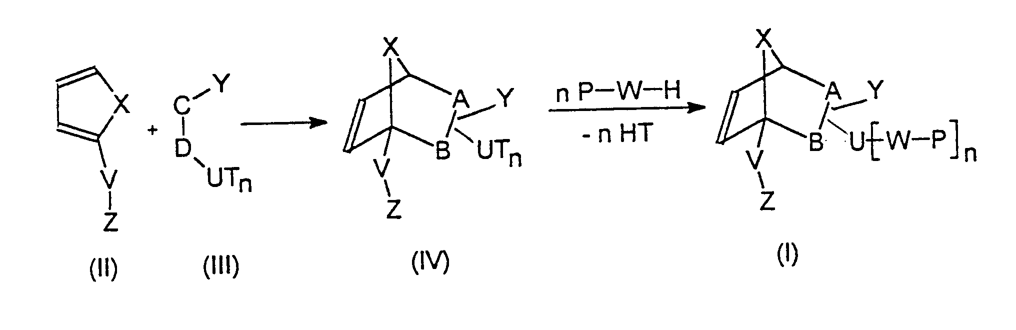

Dabei sind
C-D = C=C oder C≡C;
H = Wasserstoff; und
T = Halogen, OH oder OR
und die übrigen Variablen so wie vorstehend definiert.

Die eingesetzte substituierte Dien(meth)acrylverbindung (II) ist allgemein durch Umsetzung von geeignet substituierten Furanen (X = O) oder Cyclopentadienen (X = CH₂) mit einer entsprechenden (Meth)acrylverbindung gemäß nachstehender Reaktionsgleichung zugänglich:

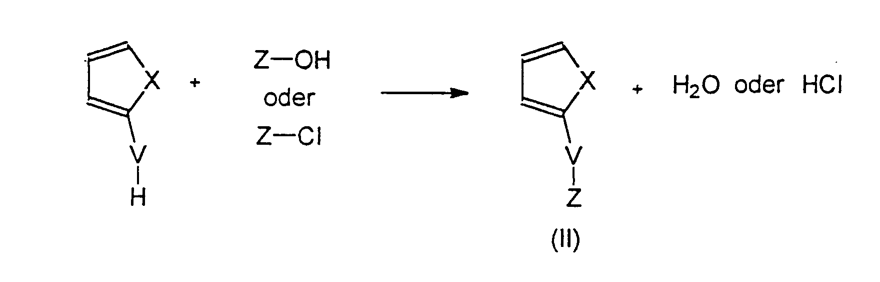

Als Dienophile (Y-C-D-UTₙ) eignen sich insbesondere Derivate der Maleinsäure oder Acetylendicarbonsäure, z.B. Maleinsäure-2-hydroxyethylmonoesteroderAcetylendicarbonsäure-2-hydroxyethylmonoester.

So kann z.B. zur Herstellung von erfindungsgemäßen Hybridmonomeren, bei denen X = O ist, Furfuryl(meth)acrylat eingesetzt werden, welches einfach durch Veresterung von Furfurylalkohol mit (Meth)acrylsäurechlorid oder -anhydrid oder durch Umesterung von Furfurylalkohol mit Methyl(meth)acrylat zugänglich ist. Das Furfuryl(meth)acrylat wird dann z.B. mit Maleinsäureanhydrid im Rahmen einer Diels-Alder-Reaktion (vgl. H. Wollweber, Diels-Alder-Reaktion, G. Thieme-Verlag 1972) zum bicyclischen Anhydrid (**A**) umgesetzt, welches den chemischen Namen exo-1-(Methacryloyloxymethyl)-7-oxabicyclo[2.2.1]hept-5-en-2,3-dicarbonsäureanhydrid hat. Durch weitere Reaktion von (**A**) mit einem polymerisierbaren Alkohol, wie 4-Hydroxystyrol, Ethylenglykolmonovinylether, Allylalkohol oder 2-Hydroxyethyl(meth)acrylat, wird schließlich in einfacher Weise das entsprechende Hybridmonomer erhalten. Diese Reaktionsfolge ist nachstehend am Beispiel der Umsetzung des bicyclischen Anhydrids (**A**) mit 4-Hydroxystyrol erläutert:

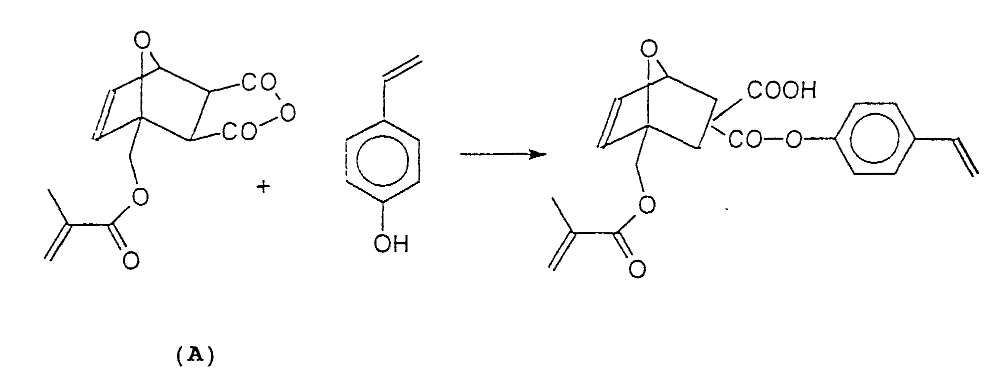

Die polymerisierbaren Gruppen der erfindungsgemäßen Hybridmonomere können nach verschiedenen Mechanismen polymerisiert werden, so daß eine mehrstufige Polymerisation, die Kombination von verschiedenen Polymerisationsmechanismen oder die Synthese von polymerisierbaren Polymeren möglich ist.

So lassen sich 7-Oxa-bicyclo[2.2.1]heptenyl-Gruppen der erfindungsgemäßen Monomeren unter den aus der Literatur bekannten Bedingungen (vgl. z.B. S.-Y. Lu et al. in Macromol. Chem. Phys. **195** (1994) 1273) z.B. in Gegenwart von käuflichem Ruthenium(III)chlorid in wäßrig-alkoholischem Medium durch eine ringöffnende Metathesepolymerisation in Polymere überführen. Die (Meth)acrylatgruppen können nach bekannten Methoden der radikalischen Polymerisation (vgl. P. Rempp, E.W. Merill in Polymer Synthesis, Hüthig & Wepf Verlag, Basel 1986, S. 91 ff. und 114 ff.) polymerisiert werden, wobei eine Acrylatgruppe in Gegenwart einer Methacrylatgruppe selektiv mit ZnBr₂ als Katalysator durch die Technik der Gruppentransfer-Polymerisation (GTP) (vgl. D.Y. Sogah, W.R. Hertler, O.W. Webster, G.M. Cohen in Macromolecules **20** (1987) 1473) polymerisiert werden kann.

Weitere polymerisierbare Gruppen können dann nach einem anderen Mechanismus polymerisiert werden, was nachstehend anhand einiger bevorzugter erfindungsgemäßer Hybridmonomere erläutert wird.

Bei dem erfindungsgemäßen Hybridmonomer (**1**) ist A-B = C-C, X = O, V = CH₂O, U = -COO(CH₂)₂-, W = entfällt, n = 1, Z = Methacryl-Rest, Y = COOH und P = Styryl-Rest. Dieses Hybridmonomer enthält drei unterschiedlich polymerisierbare Gruppen, nämlich (1.) die 7-Oxa-bicyclo[2.2.1.]hept-2-enyl-Gruppe, die einer ringöffnenden Metathesepolymerisation zugänglich ist, (2.) die Styrylgruppe, die sich kationisch homo- oder copolymerisieren läßt und (3.) die Methacrylatgruppe, die radikalisch polymerisiert oder copolymerisiert werden kann.

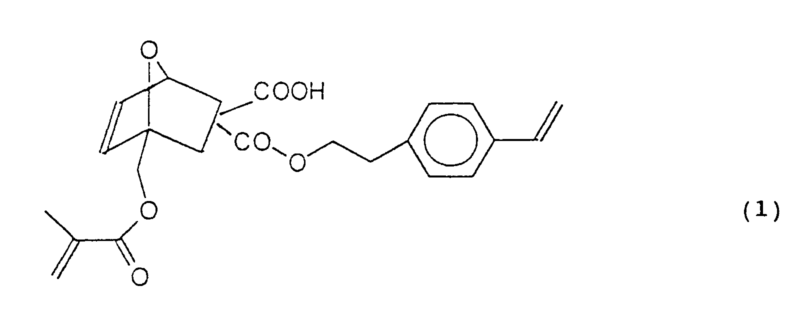

Ein weiteres Beispiel ist das erfindungsgemäße Hybridmonomer (**2**), bei dem A-B = C-C, X = O, V = CH₂O, U = -COO(CH₂)₂-, W = entfällt, n = 1, Z = Methacryl-Rest, Y = COOH und P = Vinylether-Rest. Dieses Hybridmonomer enthält ebenfalls drei unterschiedlich polymerisierbare Gruppen und kann zunächst durch eine ringöffnende Metathesepolymerisation der 7-Oxa-bicyclo[2.2.1.]hept-2-enyl-Gruppe in ein Polymer überführt werden, welches dann in zwei weiteren Schritten, z.B. zunächst radikalisch über die Methacryl-Gruppe und dann kationisch über die Vinylethergruppe, vernetzbar ist.

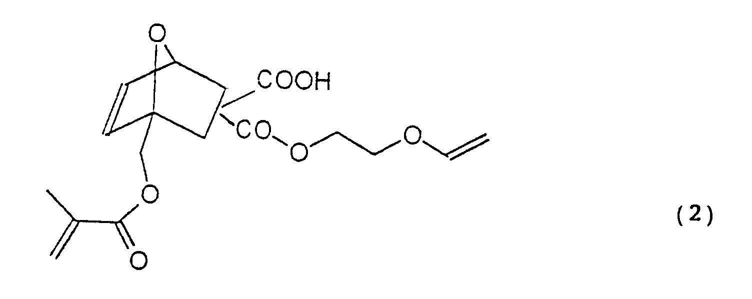

Ein weiteres Beispiel stellt schließlich das erfindungsgemäße Hybridmonomer (**3**) dar, bei dem A-B = C-C, X = O, V = CH₂O, U =-COO(CH₂)₂-O, W = entfällt, n = 1, Z = Methacryl-Rest, Y = COOH, P = Acryl-Gruppe. Die Acrylatgruppe dieses Hybridmonomers kann zunächst nach Schutz der Carboxylgruppe, z.B. mit einem Trimethylsilyl-Rest, durch Anwendung der Technik der Gruppentransferpolymerisation (GTP) selektiv polymerisiert werden. Die entstandenen polymergebundenen Methacrylatgruppen lassen sich dann in einem zweiten Schritt z.B. in Lösung mit anderen Methacrylaten copolymerisieren, d.h. zur Bildung von entsprechenden Pfropfästen einsetzen. Das erhaltene Pfropfcopolymerisat kann schließlich noch über die vorhandenen Norbornenyl-Gruppen vernetzt werden.

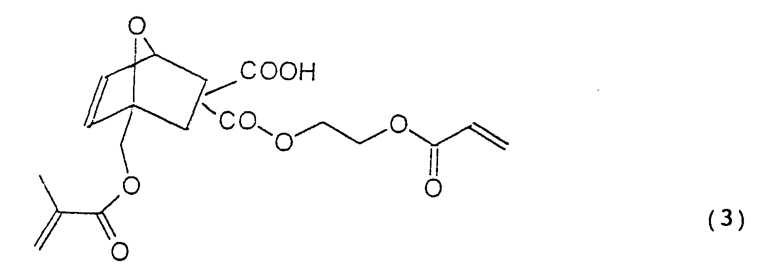

Die Strukturformeln von weiteren bevorzugten Hybridmonomeren (**4**) bis (**6**) sind im folgenden aufgeführt.

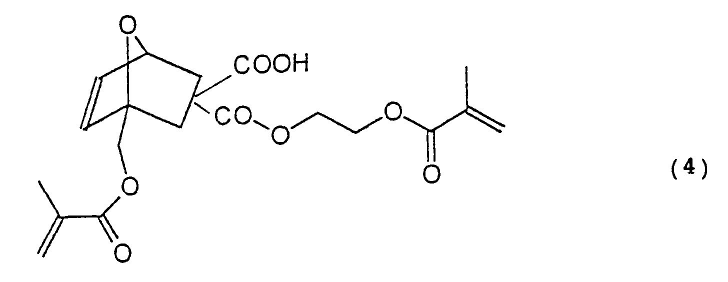

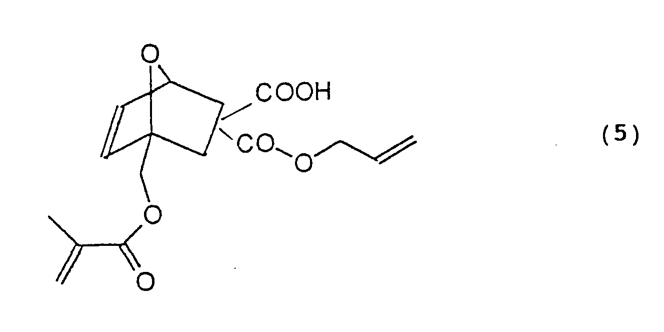

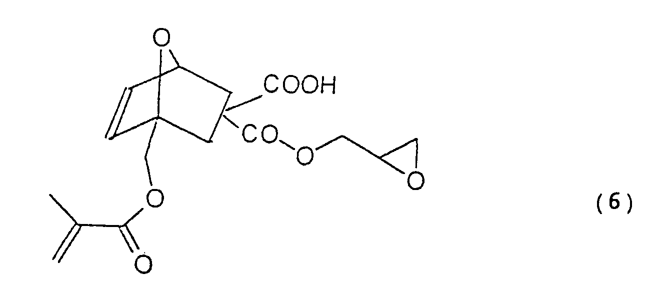

Aufgrund des Vorhandenseins von polymerisierbaren Gruppen eignen sich die erfindungsgemäßen Hybridmonomere als Ausgangsmaterialien für die Herstellung von Polymeren und Copolymeren. Dabei können sie in Abhängigkeit von der Art der polymerisierbaren Gruppen und unter Beachtung möglicher Nebenreaktionen durch vorhandene weitere funktionelle Gruppen mittels bekannter Methoden der radikalischen oder ionischen Polymerisation oder der ringöffnenden Metathesepolymerisation stufenweise homopolymerisiert oder zusammen mit geeigneten Comonomeren copolymerisiert werden.

Darüber hinaus können die erfindungsgemäßen Hybridmonomere und daraus erhaltene Polymere und Copolymere über verbleibende polymerisierbare Gruppen funktionalisiert werden, wie z.B. durch Addition von SH- oder NH-Gruppen von gewünscht funktionalisierten Verbindungen an die verbleibenden polymerisierbaren Gruppen. So kann z.B. das Hybridmonomer (**2**) zunächst radikalisch polymerisiert werden, wobei nur die Methacrylat-Gruppen verbraucht werden. An die verbleibenden Doppelbindungen kann dann z.B. das käufliche 3-Mercaptopropyltrimethoxysilan addiert werden.

Die erfindungsgemäßen Hybridmonomere können dementsprechend sowohl zur Herstellung von stufenweise polymerisierbaren Homopolymeren als auch als Comonomere eingesetzt werden, wobei weitere Reaktionen, wie Vernetzungs- oder Kopplungsreaktionen oder Copolymerisationen, der gebildeten Copolymere aufgrund vorhandener weiterer polymerisierbarer Gruppen möglich sind. Darüber hinaus gestatten die erfindungsgemäßen Hybridmonomere die Kombination von Monomerbausteinen von anionisch oder radikalisch polymerisierbaren (Meth)acrylaten mit den kationisch polymerisierbaren Vinylethern. Schließlich können zusätzliche funktionelle Gruppen der erfindungsgemäßen Monomere, wie z.B. saure Carboxyl- oder Phosphonsäuregruppen, bei Verwendung der Monomere in Adhäsiven vorteilhaft sein, da sie zur Erhöhung der Haftung der Adhäsive an verschiedenen Substraten, wie z.B. Kunststoff oder Metall, beitragen können.

Sofern gewünscht, können die erfindungsgemäßen Hybridmonomere oder daraus erhaltene Polymere durch Zugabe von Additiven, wie Füllstoffen, Pigmenten, Weichmachern und Stabilisatoren, modifiziert werden.

Die erfindungsgemäßen Hybridmonomere können als Bestandteil von durch Polymerisation härtbaren ungefüllten Monomermischungen, z.B. für Klebstoffe oder Adhäsive, oder von gefüllten, durch Polymerisation härtbaren Massen, z.B. für die Herstellung von Kompositematerialien, Anwendung finden. Dabei erweist es sich als Vorteil, das die Hybridmonomere eine Kombination von nach verschiedenen Mechanismus polymerisierenden Monomeren gestatten.

Bevorzugt werden die erfindungsgemäßen Hybridmonomere als Dentalmaterial oder Bestandteil von Dentalmaterial, insbesondere als Bestandteil von Dentinadhäsiven eingesetzt, wobei sich neben einer haftverbessernden Wirkung ihre Fähigkeit als vorteilhaft erweist, daß sie sich bei Raumtemperatur radikalisch härten lassen.

Besonders bevorzugt werden die erfindungsgemäßen Hybridmonomere als Bestandteil von Dentalmaterialien in einer Menge von 0,1 bis 60, insbesondere 5,0 bis 45 Gew.-%, eingesetzt.

Bei Verwendung der erfindungsgemäßen Hybridmonomere als Bestandteil von Dentalmaterialien werden sie vorteilhafterweise mit polymerisierbaren organischen Bindemitteln, Füllstoffen, Initiatoren und/oder weiteren Zusätzen, wie üblichen Stabilisatoren, z.B. Hydrochinonmonomethylether (MEHQ) oder 2,6-Di-tert.-butyl-4-methylphenol (BHT), UV-Absorbern, Pigmenten, Farbstoffen oder Lösungsmitteln, kombiniert.

Als polymerisierbare organische Bindemittel eignen sich alle für einen Dentalwerkstoff brauchbaren Bindemittel, insbesondere monofuktionelle oder polyfunktionelle Methacrylate, die allein oder in Mischungen eingesetzt werden können. Bevorzugte Beispiele für diese Verbindungen sind Methylmethacrylat, Isobutylmethacrylat, Cyclohexylmethacrylat, Tetraethylenglycoldimethacrylat, Triethylenglycoldimethacrylat, Diethylenglycoldimethyacrylat, Ethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, 2,2-Bis-4-(3-methacryloxy-2-hydroxy-propoxy)-phenylpropan (Bis-GMA) sowie die Produkte der Reaktion von Isocyanaten, insbesondere Di- und/oder Triisocyanaten, mit OH-gruppenhaltigen Methacrylaten. Besonders bevorzugte Beispiele für die zuletzt genannten Produkte sind durch Reaktion von 1 Mol Hexamethylendiisocyanat mit 2 Mol 2-Hydroxyethylenmethacrylat, von 1 Mol Tri-(6-isocyanatohexyl)biuret mit 3 Mol 2-Hydroxyethylmethacrylat und von 1 Mol 2,2,4-Trimethylhexamethylendiisocyanat mit 2 Mol 2-Hydroxyethylmethacrylat erhältlich.

Diese organischen Bindemittel werden üblicherweise in einer Menge von 0,1 bis 60 Gew.-% in dem erfindungsgemäßen Dentalmaterial eingesetzt.

Beispiele für bevorzugte Füllstoffe sind Quarz-, Glaskeramik- und Glaspulver, insbesondere Bariumsilikatgläser, Li/Al-Silikatgläser und Bariumgläser, Aluminium- oder Siliciumoxide, feinstteilige Kieselsäuren, insbesondere pyrogene oder gefällte Kieselsäuren, röntgenopake Füllstoffe, wie Ytterbiumtrifluorid.

Die Füllstoffe werden typischerweise in einer Menge von 0 bis 80 Gew.-% eingesetzt.

Die erfindungsgemäßen Dentalmaterialien können heiß, kalt oder durch Licht polymerisiert werden. Als Initiatoren für die Heißpolymerisation können die bekannten Peroxide wie Dibenzoylperoxid, Dilauroylperoxid, tert.-Butylperoctoat oder tert.-Butylperbenzoat eingesetzt werden. Darüber hinaus sind auch 2,2'-Azoisobuttersäurenitril (AIBN), Benzpinakol und 2,2'-Dialkylbenzpinakole geeignet.

Als Initiatoren für die Photopolymerisation können zum Beispiel Benzophenon und seine Derivate sowie Benzoin und seine Derivate verwendet werden. Weitere bevorzugte Photoinitiatoren sind die α-Diketone wie 9,10-Phenanthrenchinon, Diacetyl, Furil, Anisil, 4,4'-Dichlorbenzil und 4,4'-Dialkoxybenzil. Besonders bevorzugt wird Campherchinon verwendet. Darüber hinaus eignet sich auch die Gruppe der Acylphosphinoxide gut zur Initiierung der Photopolymerisation. Zur Beschleunigung der Initiierung werden die Photoinitiatoren vorzugsweise zusammen mit einem Reduktionsmittel, besonders bevorzugt mit einem Amin, insbesondere einem aromatischen Amin, eingesetzt.

Als Initiatoren für die Kaltpolymerisation werden Radikale liefernde Redox-Systeme, zum Beispiel Benzoyl- bzw. Lauroylperoxid zusammen mit Aminen wie N,N-Dimethyl-p-toluidin, N,N-Dihydroxyethyl-p-toluidin oder anderen strukturverwandten Aminen eingesetzt.

Speziell bei Dentalmaterialien zur Zementierung von Dentalrestaurationen, wie beispielsweise Glaskeramik-Inlays, -Onlays, -Teilkronen und -Kronen, hat sich die Kombination von Photoinitiatoren mit unterschiedlichen Redoxsystemen bewährt. Bevorzugt sind Kombinationen aus Campherchinon, Benzoylperoxid und Aminen wie beispielsweise N,N-Dimethyl-p-toluidin und/oder N,N-Cyanoethylmethylanilin.

Die Konzentration der Initiatoren liegt bevorzugt im Bereich von 0,05 bis 1,5 Gew.-%, besonders bevorzugt im Bereich von 0,2 bis 0,8 Gew.-%, bezogen auf die Menge der eingesetzten Monomeren.

Die Erfindung wird im folgenden anhand von Beispielen näher erläutert.

### Beispiele

### Beispiel 1 Synthese des bicyclischen Anhydrids (A) (exo-1-(Methacryloyloxymethyl)-7-oxabicyclo[2.2.1]hept-5-en-2,3-dicarbonsäureanhydrid)

In einem 500 ml Dreihalskolben mit mechanischem Rührer, Thermometer und Calciumchlorid-Trockenrohr wurden 1 mol (166 g) Furfurylmethacrylat und 1,1 mol (107,8 g) Maleinsäureanhydrid zusammen mit 10 mg Hydrochinonmonomethylether (MEHQ) in 250 ml über Molekularsieb getrocknetem Butylacetat suspendiert und 2 Tage bei Raumtemperatur gerührt. Die anfänglich trübe Dispersion wurde nach ca. 1 Stunde klar, und nach etwa einem Tag bildete sich das Produkt als fester Niederschlag (ca. 130 g), der abfiltriert, mit Butylacetat gewaschen und im Vakuum bis zur Gewichtskonstanz getrocknet wurde. Aus dem Filtrat konnte nach einigen Tagen weiteres Produkt isoliert werden, so daß insgesamt 188 g (70 % Ausbeute) einer nahezu farblosen kristallinen Substanz erhalten wurden. Das Anhydrid A dient als Ausgansmaterial für die Herstellung erfindungsgemäßer Monomere.

| | | | |
|---|---|---|---|
| Elementaranalyse (C₁₃H₁₂O₆) | Ber. | C 59,08 | H 4,58 |
| | Gef. | C 58,89 | H 4,61 |

¹H-NMR (CDCl₃, ppm) : 1,91 (s, 3H, CH₃), 3,42 und 3.52 (d, 2H, CH-2,3-exo), 4,55 und 4,83 (d, 2H, CH₂O), 5,33 (s, 1H, -CH<), 5,67 und 6.04 (s, 2H, CH₂=), 6,52 (d, 2H, -CH=).

IR (KBr, cm⁻¹): 1630 (C=C), 1720 (C=O-Ester), 1780 und 1860 (C=O-Anhydrid).

### Beispiel 2: Synthese des exo-1-[(Methacryloyloxy)methyl]-7-oxabicyclo[2.2.1]hept-5-en-2,3-dicarbonsäuremono allylesters (5)

In einem 100 ml Zweihalskolben wurden unter Rühren 41 mmol (2,32 g) Allylalkohol und 10 mg Phenothiazin in 50 ml wasserfreiem Tetrahydrofuran (THF) gelöst, und in dieser Lösung wurden anschließend 40 mmol (10,6 g) bicyclisches Anhydrid (**A**) suspendiert, welches gemäß Beispiel 1 hergestellt worden war. Nach Abkühlen auf 10°C wurde eine Lösung von 40 mmol (4,0 g) Triethylamin (TEA) und 0,25 g 4-Dimethylamino-pyridin (DMAP) in 10 ml THF innerhalb von 20 Minuten zugetropft. Nach Erwärmung auf Raumtemperatur wurde noch 2 Stunden zur Vervollständigung der Reaktion gerührt. Das Reaktionsgemisch wurde in einem Gemisch aus 100 ml 10 %iger wäßriger NaHCO₃-Lösung und 50 ml Methylenchlorid aufgenommen. Die wäßrige Phase wurde abgetrennt und mit Methylenchlorid ausgeschüttelt, mit konzentrierter Salzsäure auf pH = 1-2 eingestellt und erneut mit Methylenchlorid extrahiert. Dann wurden die vereinigten Methylenchlorid-Phasen über wasserfreiem Na₂SO₄ getrocknet und im Vakuum bei einer Temperatur von unter 25°C vom Lösungsmittel befreit. Der anfallende feste Rückstand wurde in Methylenchlorid gelöst und unter Zusatz von Petrolether ausgefällt. Nach Abfiltrieren und Trocknen wurden 4,3 g (33 % Ausbeute) weiße Kristalle erhalten.

| | | | |
|---|---|---|---|
| Elementaranalyse (C₁₆H₁₈O₇): | Ber.: | C 59,63 | H 5,63 |
| | Gef.: | C 59,50 | H 5,59 |

¹H-NMR (CDCl₃, ppm): 1,95 (s, 3H, CH₃); 3,05 (m, 2H, H-2,3); 4,6 (d, 2H, COOCH₂); 4,75 (d, 2H, CH₂, CH₂-O-Methacryl); 5,2 (m), 5,35 (d) und 6,0 (m, 3H, CH=CH₂-Allyl); 5,35 (d, 1H, H-4); 5,6 und 6,2 (s, 2H, CH₂=Methacrylat); 6,45 (m, 2H, H-5,6); 8,85 (s, 1H, COOH).

IR (KBr, cm⁻¹): 709 (m), 815 (w), 870 (m), 934 (s), 989 (m), 1108 (w), 1177 (s), 1297 (s), 1422 (m), 1638 (m), 1718 (s), 2957 (m).

### Beispiel 3 Synthese des exo-1-[(Methacryloyloxy)methyl]-7-oxabicyclo[2.2.1]hept-5-en-2,3-dicarbonsäure mono[(2-methacryloyloxy)ethyl]esters (4)

In einem 100 ml Zweihalskolben mit Magnetrührer und Trockenrohr wurden 41 mmol (5,33 g) 2-Hydroxyethylmethacrylat (HEMA) und 0,01 g MEHQ in 50 ml THF gelöst, und zu dieser Lösung wurden 40 mmol (10,6 g) bicyclisches Anhydrid (**A**) gegeben. Unter Rühren und Eiskühlung (T < 10°C) wurde eine Lösung von 40 mmol (4,0 g) Triethylamin und 0,25 g DMAP in 10 ml THF langsam zugetropft. Nach vollständiger Zugabe wurde die weiße Suspension klar und cremefarben. Die Reaktion wurde nach 30 Minuten abgebrochen, indem der Ansatz in 50 ml Methylenchlorid aufgenommen und die erhaltene Mischung mit 120 ml 10 %iger wäßriger NaHCO₃-Lösung versetzt wurde. Die organische Phase wurde abgetrennt. Die wäßrige Phase wurde nochmals mit 50 ml Methylenchlorid extrahiert und mit konzentrierter Salzsäure unter Kühlung auf einen pH ≈ 2 eingestellt. Dabei schied sich eine dicke weiße Flüssigkeit ab, die in 300 ml Methylenchlorid aufgenommen wurde. Die wäßrige Phase wurde abgetrennt und dreimal mit je 150 ml Methylenchlorid extrahiert. Anschließend wurden die vereinigten organischen Phasen mit etwas 2,6-Di-tert.-butyl-4-methylphenol (BHT) stabilisiert und über Na₂SO₄ getrocknet. Nach Abdestillieren des Lösungsmittels im Vakuum bei einer Temperatur von unter 25 °C wurde ein gelbes Öl erhalten. Nach Trocknung im Feinvakuum ergab sich eine Ausbeute an (**4**) von 6,6 g (42 %), wobei das Produkt ca. 7 % HEMA enthielt.

| | | | |
|---|---|---|---|
| Elementaranalyse (C₁₉H₂₂O₉) | Ber. | C 57,87 | H 5,62 |
| | Gef. | C 56,32 | H 5,72 |

¹H-NMR (CDCl₃, ppm): 1,95 (s, 3H, CH₃); 3,0 (m, 2H, H-2,3); 4,4 (s, 2 × 2H, COOCH₂); 4,75 (m, 2H, CH₂-O-Methacryl); 5,4 (s, 1H, H-4); 5,6 und 6,2 (s, 2 × 2H, CH₂=Methacryl); 6,45 (m, 2H, H-5,6); 8,8 (s, 1H, COOH).

IR (KBr, cm⁻¹): 733 (w), 815 (w), 939 (m), 1164 (s), 1298 (m), 1453 (w), 1636 (m), 1721 (s), 2960 (m).

### Beispiel 4: Synthese des exo-1-[(Methacryloyloxy)methyl]-7-oxabicyclo[2.2.1]hept-5-en-2,3-dicarbonsäure -mono[(2-acryloyloxy)ethyl]esters (3)

In einem 100 ml Zweihalskolben mit Magnetrührer und Trockenrohr wurden 41 mmol (4,93 g) 2-Hydroxyethylacrylat (HEA) und 0,01 g MEHQ in 50 ml THF gelöst, und zu dieser Lösung wurden 40 mmol (10,6 g) bicyclisches Anhydrid (**A**) gegeben. Unter Rühren und Eiskühlung (T < 10 °C) wurde eine Lösung von 40 mmol (4,0 g) TEA und 0,25 g DMAP in 10 ml THF langsam zugetropft. Nach vollständiger Zugabe wurde die weiße Suspension klar und cremefarben. Die Reaktion wurde nach 30 Minuten abgebrochen, indem der Ansatz in 50 ml Methylenchlorid aufgenommen und die erhaltene Mischung mit 120 ml 10 %iger wäßriger NaHCO₃-Lösung versetzt wurde. Die organische Phase wurde abgetrennt. Die wäßrige Phase wurde nochmals mit 50 ml Methylenchlorid extrahiert und mit konzentrierter Salzsäure unter Kühlung auf einen pH ≈ 2 eingestellt. Dabei schied sich eine dicke weiße Flüssigkeit ab, die in 300 ml Methylenchlorid aufgenommen wurde. Die wäßrige Phase wurde abgetrennt und dreimal mit je 150 ml Methylenchlorid extrahiert. Anschließend wurden die vereinigten organischen Phasen mit BHT stabilisiert und über Na₂SO₄ getrocknet. Nach Abdestillieren des Lösungsmittels im Vakuum bei einer Temperatur von unter 25 °C und Trocknen im Feinvakuum erhielt man (**3**) als ein gelbes Öl in 65 %iger Ausbeute (9,9 g), wobei das Produkt noch ca. 5 % HEA enthielt.

| | | | |
|---|---|---|---|
| Elementaranalyse (C₁₈H₂₀O₉) | Ber. | C 56,85 | H 5,30 |
| | Gef. | C 54,60 | H 5,35 |

¹H-NMR (CDCl₃, ppm): 1,95 (s, 3H, CH₃); 3,1 (m, 2H, H-2,3); 4,4 (s, 2 × 2H, COOCH₂); 4,75 (d, 2H, CH₂-O-Methacryl); 5,4 (s, 1H, H-4); 5,6 bis 6,2 (m, 5H, CH₂=Methacryl, CH₂=CH-Acryl); 6,45 (m, 2H, H-5,6); 10,25 (s, 1H, COOH).

IR (KBr, cm⁻¹): 733 (w), 812 (m), 934 (m), 987 (m), 1075 (w), 1168 (s), 1298 (m), 1410 (m), 1636 (m), 1730 (s), 2960 (w).

### Beispiel 5: Synthese des exo-1-[(Methacryloyloxy)methyl]-7-oxabicyclo[2.2.1]hept-5-en-2,3-dicarbonsäure -mono[(2-vinyloxy)ethyl]esters (2)

In einem 100 ml Zweihalskolben mit Magnetrührer und Trockenrohr wurden 41 mmol (3,5 g) 2-Hydroxyethylvinylether und 0,01 g MEHQ in 50 ml THF gelöst, und zu dieser Lösung wurden 40 mmol (10,6 g) bicyclisches Anhydrid (**A**) gegeben. Unter Rühren und Eiskühlung (T < 10 °C) wurde eine Lösung von 40 mmol (4,0 g) Triethylamin und 0,25 g DMAP in 10 ml THF langsam zugetropft. Nach vollständiger Zugabe wurde die erhaltene Suspension klar und cremefarben. Die Reaktion wurde nach 30 Minuten abgebrochen, indem der Ansatz in 50 ml Methylenchlorid aufgenommen und die erhaltene Mischung mit 120 ml 10 %iger wäßriger NaHCO₃-Lösung versetzt wurde. Die organische Phase wurde abgetrennt. Die wäßrige Phase wurde nochmals mit 50 ml Methylenchlorid extrahiert und mit konzentrierter Salzsäure unter Kühlung auf einen pH ≈ 2 eingestellt. Dabei schied sich eine dicke weiße Flüssigkeit ab, die in 300 ml Methylenchlorid aufgenommen wurde. Die wäßrige Phase wurde dreimal mit je 150 ml Methylenchlorid extrahiert. Anschließend wurden die vereinigten organischen Phasen mit BHT stabilisiert und über Na₂SO₄ getrocknet. Nach Abdestillieren des Lösungsmittels bei einer Temperatur von unter 25°C und Trocknen im Feinvakuum erhielt man (**2**) als ein gelbliches Öl in 38,5 %iger Ausbeute (5,4 g).

¹H-NMR (CDCl₃, ppm): 1,95 (s, 3H, CH₃); 3,0 (m, 2H, H-2,3); 3,7 (2H, CH₂-O); 4,25 (m, 2 × 2H, COOCH₂ und CH₂=); 4,75 (d, 2H, CH₂-O-Methacryl); CH₂=Methacryl); 5,4 (d, 1H, H-4); 5,6 und 6,2 (s, 2H, 6,5 (m, 2H, H-5,6 und 1H, O-CH=); 8,6 (s, 1H, COOH).

IR (KBr, cm⁻¹): 715 (w), 814 (w), 934 (w), 1077 (w), 1164 (s), 1299 (m), 1635 (w), 1723 (s), 2959 (w).

### Beispiel 6: Synthese des exo-1-[(Methacryloyloxy)methyl]-7-oxabicyclo[2.2.1]hept-5-en-2,3-dicarbonsäure -mono[(1,3-dimethacryloyloxy)propyl]esters (7)

In einem 100 ml Zweihalskolben mit Magnetrührer und Trockenrohr wurden 41 mmol (9,12 g) Glycerindimethacrylat und 0,01 g MEHQ in 50 ml THF gelöst, und zu dieser Lösung wurden 40 mmol (10,6 g) bicyclisches Anhydrid (**A**) gegeben. Unter Rühren und Eiskühlung (T < 10 °C) wurde eine Lösung von 40 mmol (4,0 g) TEA und 0,25 g DMAP in 10 ml THF langsam zugetropft. Nach vollständiger Zugabe wurde die weiße Suspension klar und cremefarben. Die Reaktion wurde nach 30 Minuten abgebrochen, indem der Ansatz in 50 ml Methylenchlorid aufgenommen und die erhaltene Mischung mit 120 ml 10 %iger wäßriger NaHCO₃-Lösung versetzt wurde. Die organische Phase wurde abgetrennt. Die wäßrige Phase wurde nochmals mit 50 ml Methylenchlorid extrahiert und mit konzentrierter Salzsäure unter Kühlung auf einen pH ≈ 2 eingestellt. Dabei schied sich eine dicke weiße Flüssigkeit ab, die in 300 ml Methylenchlorid aufgenommen wurde. Die wäßrige Phase wurde abgetrennt und dreimal mit je 150 ml Methylenchlorid extrahiert. Anschließend wurden die vereinigten organischen Phasen mit BHT stabilisiert und über Na₂SO₄ getrocknet. Nach Abdestillieren des Lösungsmittels im Vakuum bei einer Temperatur von unter 25°C und Trocknen im Feinvakuum erhielt man (**7**) als ein viskoses Öl in 17,8 %iger Ausbeute (3,5 g).

¹H-NMR (CDCl₃, ppm): 1,95 (s, 3H, CH₃); 3,05 (m, 2H, H-2,3); 4,35 (m, 5H, CH₂CH-CH₂); 4,75 (d, 2H, CH₂-O-Methacryl); 5,45 (d, 1H, H-4); 5,6 und 6,2 (s, 2H, CH₂=Methacryl); 6,45 (m, 2H, H-5,6); 8,75 (s, H, COOH).

IR (KBr, cm⁻¹): 652 (w), 737 (w), 814 (m), 863 (w), 942 (m), 1017 (m), 1160 (s), 1296 (s), 1454 (m), 1638 (m), 1723 (s), 2960 (m).

### Beispiel 7: Synthese des exo-1-[(Methacryloyloxy)methyl]-7-oxabicyclo[2.2.1]hept-5-en-2,3-dicarbonsäure -mono(n-propyl)esters (8)

In einem 100 ml Zweihalskolben mit Magnetrührer und Trockenrohr wurden 41 mmol (2,4 g) n-Propanol und 0,01 g MEHQ in 50 ml THF gelöst, und zu dieser Lösung wurden 40 mmol (10,6 g) bicyclisches Anhydrid (**A**) gegeben. Unter Rühren und Eiskühlung (T < 10 °C) wurde eine Lösung von 40 mmol (4,0 g) Triethylamin und 0,25 g DMAP in 10 ml THF langsam zugetropft. Nach vollständiger Zugabe wurde die weiße Suspension klar und cremefarben. Die Reaktion wurde nach 30 Minuten abgebrochen, indem der Ansatz in 50 ml Methylenchlorid aufgenommen und die erhaltene Mischung mit 120 ml 10 %iger wäßriger NaHCO₃-Lösung versetzt wurde. Die organische Phase wurde abgetrennt. Die wäßrige Phase wurde nochmals mit 50 ml Methylenchlorid extrahiert und mit konzentrierter Salzsäure unter Kühlung auf einen pH ≈ 2 eingestellt. Dabei schied sich eine dicke weiße Flüssigkeit ab, die in 300 ml Methylenchlorid aufgenommen wurde. Die wäßrige Phase wurde abgetrennt und dreimal mit je 150 ml Methylenchlorid extrahiert. Anschließend wurden die vereinigten organischen Phasen mit BHT stabilisiert und über Na₂SO₄ getrocknet. Nach Abdestillieren des Lösungsmittels im Vakuum bei einer Temperatur von unter 25 °C erhielt man ein gelbes Öl, aus welchem sich beim Stehen ein weißer Feststoff abschied. Nach Auflösen des Feststoffs in Methylenchlorid bei Raumtemperatur wurde unter Zusatz von Petrolether ein weißer Niederschlag ausgefällt. Der weiße Niederschlag wurde abgesaugt, mit Petrolether gewaschen und im Feinvakuum getrocknet, wobei 3,7 g an (**8**) (29 % Ausbeute) erhalten wurden.

| | | | |
|---|---|---|---|
| Elementaranalyse (C₁₆H₂₀O₇) | Ber. | C 59 26 | H 6,17 |
| | Gef. | C 58,93 | H 6,29 |

¹H-NMR (CDCl₃, ppm): 0,95 (m, 3H, CH₃-Propyl); 1,7 (m, 2H, CH₂-Propyl); 2,0 (s, 3H, CH₃-Methacryl); 3,15 (m, 2H, H-2,3); 4,15 (m, 2H, COOCH₂); 4,75 (m, 2H, CH₂-O-Methacryl); 5,55 (d, 1H, H-H-4); 5,7 und 6,25 (s, 2H, CH₂=Methacryl); 6,55 (m, 2H, H-5,6); offset (s, 1H, COOH).

IR (KBr, cm⁻¹): 733 (s), 814 (m), 872 (m), 913 (s), 993 (m), 1111 (w), 1167 (s), 1297 (s), 1418 (m), 1455 (m), 1635 (m), 1724 (s), 2969 (m).

Die Verbindung (**8**) diente als Modellsubstanz, um die Charakterisierung der polymerisierten erfindungsgemäßen Hybridmonomere, z.B. über die Molmassen, zu erleichtern. Diese ist nicht einfach, da es zur Bildung von Netzwerkpolymeren kommt.

### Beispiel 8: Radikalische Lösungspolymerisation der Monomeren

Durch jeweiliges Auflösen der erfindungsgemäßen Hybridmonomere (**2**), (**3**), (**4**), (**5** und (**7**) sowie der als Modell fungierenden Verbindung (**8**) in Dimethylformamid (DMF) in einem Schlenkgefäß wurden Monomerlösungen mit einer Monomerkonzentration von 1,0 Mol/l hergestellt. In die jeweilige Lösung wurde Azobisisobutyronitril als Initiator in einer Menge gegeben, um eine Initiatorkonzentration von 0,02 Mol/l zu ergeben. Nach Eingabe eines mit Teflon beschichteten Magnetrührstabes wurden die Lösungen zum Entgasen in der üblichen Weise sekuriert, d.h. wiederholt unter Inertgas eingefroren und im Vakuum aufgetaut, und anschließend unter Verwendung eines Schnellbelichtungstischgerätes SUNTEST CPS (Heraeus) bei 25°C in einem thermostatierten Bad unter Rühren mit UV-Licht bestrahlt. Die Polymerisation wurde nach 1 Stunde abgebrochen, indem das Reaktionsgemisch zur Ausfällung des Polymerisats mit der 10-fachen Menge Diethylether versetzt wurde. Das durch Filtration isolierte Polymer wurde dann im Feinvakuum bis zur Gewichtskonstanz getrocknet.

Der für das jeweilige Monomer bestimmte Monomerumsatz und die zahlenmittlere Molmasse des jeweilig erhaltenen Polymeren sind in der nachfolgenden Tabelle angegeben:

| **Monomer** | **Monomerumsatz (%)** | **M**_{**n**}*** (g/mol)** |
|---|---|---|
| 2 | 43,8 | 9500 |
| 3 | 36,8 | Unlöslich, Gelzeit: 40 Minuten |
| 4 | 83,8 | Unlöslich, Gelzeit: 10 Minuten |
| 5 | 58,1 | 4960 |
| 7 | 76,1 | Unlöslich, Gelzeit: 15 Minuten |
| 8 | 51,2 | 10600 |
| Mₙ* - Zahlenmittlere Molmasse wurde durch Gelpermeationschromatographie (GPC) bei Kalibrierung mit Polymethylmethacrylat (PMMA)-Standards bestimmt | | |

### Beispiel 9: Kationische Photopolymerisation von (2) in Substanz

2 g des erfindungsgemäßen Hybridmonomers (**2**) wurden in einem Schlenkgefäß zusammen mit 1 Gew.-%, bezogen auf Menge Monomer, kationischen Photoinitator Degacure Kl 85 (Degussa) in 2 ml Methylenchlorid gelöst und homogenisiert. Anschließend wurde das Lösungsmittel im Feinvakuum entfernt, und die Probe wurde in der üblichen Weise entgast. Unter Verwendung des Schnellbelichtungstischgerätes SUNTEST CPS (Heraeus) wurde die Probe bei 25°C in einem thermostatierten Bad mit UV-Licht 20 Minuten lang bestrahlt. Unumgesetztes Monomer wurde durch Extraktion mit Diethylether von dem erhaltenen vernetzten Polymerisat abgetrennt. Dann wurde das Polymerisat im Feinvakuum bis zur Gewichtskonstanz getrocknet. Der Monomerumsatz betrug 37,5 %.

### Beispiel 10: Dentinadhäsiv auf der Basis von Hybridmonomer (5)

Auf der Basis von Hybridmonomer (**5**), dessen Herstellung in Beispiel 2 beschrieben ist, wurde ein Dentin-Primer folgender Zusammensetzung hergestellt:

| | |
|---|---|
| Monomer (**5**) | 40,0 Gew.-% |
| Wasser, deionisiert | 40,0 Gew.-% |
| 2-Hydroxyethylmethacrylat | 20,0 Gew.-% |

Zur Ermittlung der mit dieser Primerformulierung als Komponente von einem Dentinadhäsiv erzielten Scherhaftung wurden zuerst Dentinoberflächen von extrahierten, eingebetteten Zähnen mit 500-er und 1000-er Schleifpapier plangeschliffen, und die Dentinoberfläche wurde mit Druckluft getrocknet. Anschließend wurde die Formulierung aufgetragen und nach 30 Sekunden wurde die Oberfläche abgeblasen. Danach wurde Syntac-Adhäsiv-Komponente (Vivadent Ets., Liechtenstein) aufgetragen und mit einem Luftbläser verteilt. Schließlich wurde lichthärtendes Bonding, nämlich Heliobond (Vivadent Ets., Liechtenstein), aufgetragen, und es wurde belichtet. Dann wurde das Füllungskomposit Tetric (Vivadent Ets., Liechtenstein) in 2 Schichten aufgetragen und jeweils 40 Sekunden lang belichtet. Danach wurden die erhaltenen Prüfkörper in destilliertes Wasser gelegt und dort 25 Stunden lang bei 37°C gelagert. Die Bestimmung der Scherhaftfestigkeit erfolgte gemäß des ISO-Vorschlages ISO-TR 11405: "Dental material - Guidance on testing of adhesion to tooth structure", und ergab einen Wert von 8 ± 6,4 MPa bestimmt. Eine analoge Rezeptur, bei der das Monomer (**5**) durch weiteres 2-Hdroxyethylmethacrylat ersetzt wurde, ergab nur einen Wert von 2 ± 2,2 MPa.

## Patentansprüche

1. Polymerisierbare Hybridmonomere der folgenden Formel (I), dazu stereoisomere Verbindungen und Mischungen von diesen 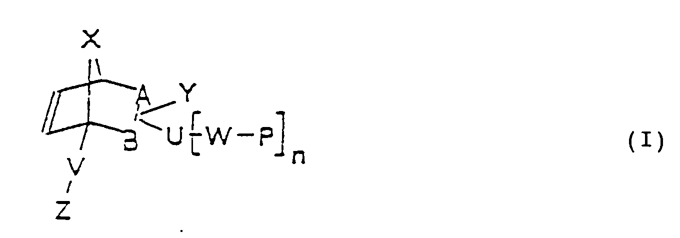 wobei A-B, X, Z, V, Y, R, U, R¹, W, P und n unabhängig voneinander die folgenden Bedeutungen haben:
A-B = C-C oder C=C;
X = CH₂ oder O;
Z = CH₂=CH-CO- oder CH₂=C(CH₃)-CO-;
V = CH₂-O oder CH₂-NH;
Y = H, substituiertes oder unsubstituiertes C₁- bis C₁₂-Alkyl, substituiertes oder unsubstituiertes C₆-bis C₁₄-Aryl, Halogen, NO₂, NH₂, NR₂, OH, OR, CN, CHO, CO-R, COOH, CO-NH₂, CO-OR, CH₂=CH-, CH₂=CH-CO-, CH₂=C(CH₃)-CO-, SH oder S-R,
wobei
R = substituiertes oder unsubstituiertes C₁-bis C₁₂-Alkyl oder substituiertes oder unsubstituiertes C₆- bis C₁₄-Aryl;
U = CO-R¹, CO-NHR¹, CO-OR¹, O-CO-NHR¹, NH-CO-OR¹, O-R¹, S-R¹ oder entfällt,
wobei
R¹ = C₁- bis C₅-Alkylen oder -Oxyalkylen oder C₆- bis C₁₂-Arylen;
W = O, NH, CO-O, CO-NH, O-CO-NH oder entfällt;
P = eine (Meth)acryl-, Vinyl-, Allyl-, Allylether-, Vinylether, Epoxy- oder Styryl-Gruppe; und
n = 1 bis 4,
wobei die bei den Resten Y und R gegebenenfalls vorhandenen Substituenten COOH, OH, Halogen, C₁- bis C₁₂-Alkoxy, -N⁺-(C₁-bis C₁₂-Alkyl)₃, -O-P=O(OH)₂ und/oder -P=O(OH)₂ sind.

2. Hybridmonomere nach Anspruch 1, **dadurch gekennzeichnet,** daß die Variablen der Formel (I) unabängig voneinander die folgende Bedeutung haben:
A-B = C-C oder C=C,
X = O,
Z = CH₂=C(CH₃)-CO-,
V = CH₂-O,
Y = COOH, CN oder CO-NH₂,
R = substituiertes oder unsubstituiertes C₁- bis C₄-Alkyl,
U = CO-OR¹ oder CO-NHR¹,
R¹ = C₁- bis C₃-Alkylen,
W = O, NH, CO-O oder entfällt,
P = Vinylether-, Epoxy-, Allyl-, Styryl- oder (Meth)acryl-Gruppe, und/oder
n = 1 oder 2.

3. Hybridmonomere nach Anspruch 1 oder 2, **dadurch gekennzeichnet,** daß sie mindestens drei nach unterschiedlichen Mechanismen polymerisierbare Gruppen enthalten.

4. Verfahren zur Herstellung der polymerisierbaren Hybridmonomere gemäß einem der Ansprüche 1 bis 3, **dadurch gekennzeichnet,** daß man durch Diels-Alder-Reaktion einer substituierten Dien(meth)acryl-Verbindung der Formel (II) mit einem substituierten Dienophil der Formel (III) eine Norbornen- oder Norbornadien-Verbindung der Formel (IV) herstellt und (IV) im Rahmen einer nucleophilen Substitution mit dem polymerisationsgruppenhaltigen Edukt P-W-H umsetzt 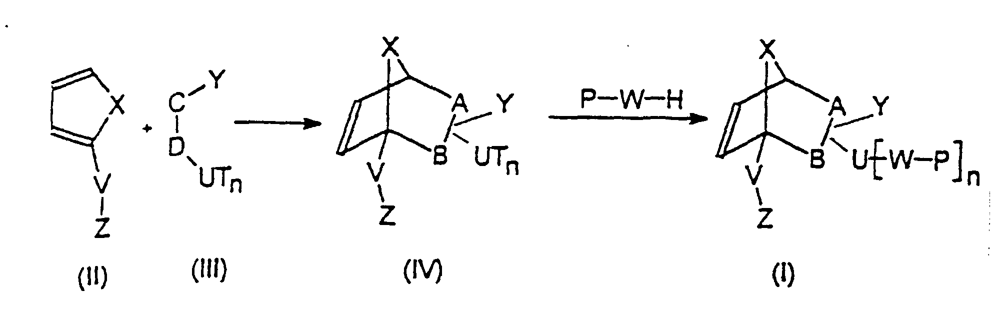 wobei
C-D = C=C oder C≡C;
H = Wasserstoff und
T = Halogen, OH oder OR
bedeuten und die übrigen Variablen so wie in Anspruch 1 definiert sind.

5. Verwendung der polymerisierbaren Hybridmonomere gemäß einem der Ansprüche 1 bis 3 als Dentalmaterial oder Bestandteil von Dentalmaterial.

6. Verwendung nach Anspruch 5, wobei das Dentalmaterial ein Dentinadhäsiv ist.

7. Dentalmaterial mit Gehalt an polymerisiertem und/oder nichtpolymerisiertem Hybridmonomer gemäß einem der Ansprüche 1 bis 3.

8. Polymere oder Copolymere, **dadurch gekennzeichnet,** daß sie durch Polymerisation oder Copolymerisation der Hybridmonomere gemäß einem der Ansprüche 1 bis 3 erhältlich sind.

## Claims

1. Polymerizable hybrid monomers of the following formula (I), stereoisomeric compounds and mixtures thereof 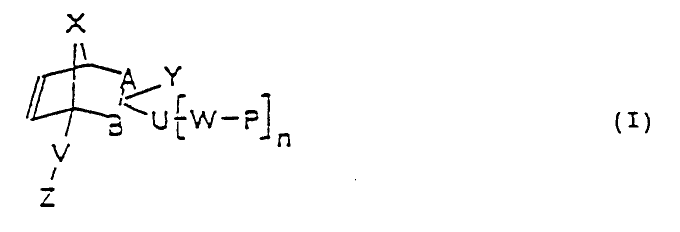 where A-B, X, Z, V, Y, R, U, R¹, W, P and n independently of one another have the following meanings:
A-B = C-C or C=C;
X = CH₂ or O;
Z = CH₂=CH-CO- or CH₂=C(CH₃)-CO-;
V = CH₂-O or CH₂-NH;
Y = H, substituted or unsubstituted C₁ to C₁₂ alkyl, substituted or unsubstituted C₆ to C₁₄ aryl, halogen, NO₂, NH₂, NR₂, OH, OR, CN, CHO, CO-R, COOH, CO-NH₂, CO-OR, CH₂=CH-, CH₂=CH-CO-, CH₂=C(CH₃)-CO-, SH or S-R,
where
R = substituted or unsubstituted C₁ to C₁₂ alkyl or substituted or unsubstituted C₆ to C₁₄ aryl;
U = CO-R¹, CO-NHR¹, CO-OR¹, O-CO-NHR¹, NH-CO-OR¹, O-R¹, S-R¹ or is absent,
where
R¹ = C₁ to C₅ alkylene or oxyalkylene or C₆ to C₁₂ arylene;
W = O, NH, CO-O, CO-NH, O-CO-NH or is absent;
P = a (meth)acrylic, vinyl, allyl, allyl ether, vinyl ether, epoxy or styryl group; and
n = 1 to 4,
where the substituents which may be present in the case of the radicals Y and R are COOH, OH, halogen, C₁ to C₁₂ alkoxy, -N⁺-(C₁ to C₁₂ alkyl)₃, -O-P=O(OH)₂ and/or -P=O(OH)₂.

2. Hybrid monomers according to Claim 1, characterized in that the variables of the formula (I) independently of one another have the following meaning:
A-B = C-C or C=C,
X = O,
Z = CH₂=C(CH₃)-CO-,
V = CH₂-O,
Y = COOH, CN or CO-NH₂,
R = substituted or unsubstituted C₁ to C₄ alkyl,
U = CO-OR¹ or CO-NHR¹,
R¹ = C₁ to C₃ alkylene,
W = O, NH, CO-O or is absent,
P = a vinyl ether, epoxy, allyl, styryl or (meth)acrylic group, and/or
n = 1 or 2.

3. Hybrid monomers according to Claim 1 or 2, characterized in that they contain at least three groups polymerizable according to different mechanisms.

4. Process for the preparation of the polymerizable hybrid monomers according to any of Claims 1 to 3, characterized in that a norbornene or norbornadiene compound of the formula (IV) is prepared by way of a Diels-Alder reaction of a substituted diene(meth)acrylic compound of the formula (II) with a substituted dienophile of the formula (III) and, by way of a nucleophilic substitution, (IV) is reacted with the polymerization group-containing reactant P-W-H 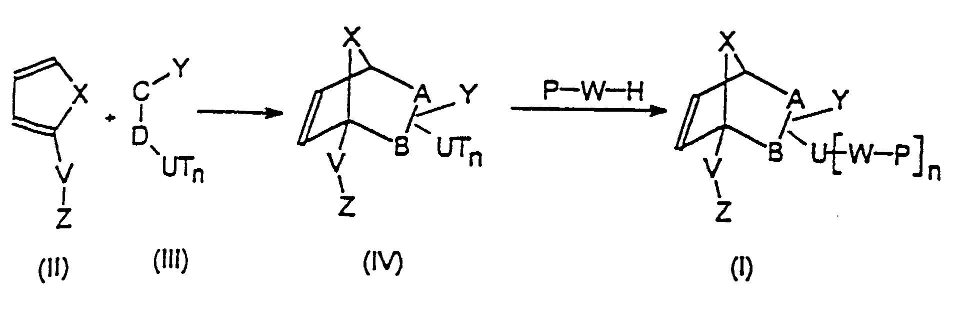 where
C-D = C=C or C≡C;
H = hydrogen and
T = halogen, OH or OR
and the remaining variables are as defined in Claim 1.

5. Use of the polymerizable hybrid monomers according to one of Claims 1 to 3 as dental material or a constituent of dental material.

6. Use according to Claim 5, for which the dental material is a dentine adhesive.

7. Dental material containing polymerized and/or nonpolymerized hybrid monomer according to any of Claims 1 to 3.

8. Polymers or copolymers characterized in that they are obtainable by polymerization or copolymerization of the hybrid monomers according to any of Claims 1 to 3.

## Revendications

1. Monomères hybrides polymérisables de formule (I) suivante, composés stéréoisomères de ceux-ci et mélanges de ceux-ci 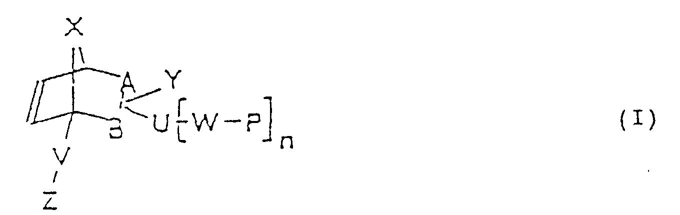 formule dans laquelle A-B, X, Z, V, Y, R, U, R¹, W, P et n ont, indépendamment les uns des autres, les significations suivantes :
A-B = C-C ou C=C ;
X = CH₂ ou O ;
Z = CH₂=CH-CO- ou CH₂=C(CH₃)-CO- ;
V = CH₂-O ou CH₂-NH ;
Y = H, un groupe alkyle en C₁-C₁₂ substitué ou non substitué, aryle en C₆-C₁₄ substitué ou non substitué, un atome d'halogène, NO₂, NH₂, NR₂, OH, OR, CN, CHO, CO-R, COOH, CO-NH₂, CO-OR, CH₂=CH-, CH₂=CH-CO-, CH₂=C(CH₃)-CO-, SH ou S-R,
R représentant un groupe alkyle en C₁-C₁₂ substitué ou non substitué ou un groupe aryle en C₆-C₁₄ substitué ou non substitué ;
U = CO-R¹, CO-NHR¹, CO-OR¹, O-CO-NHR¹, NH-CO-OR¹, O-R¹, S-R¹ ou est absent,
R¹ représentant un groupe alkylène ou oxyalkylène en C₁-C₅ ou arylène en C₆-C₁₂;
W = O, NH, CO-O, CO-NH, O-CO-NH ou est absent ;
P = un groupe (méth)acryle, vinyle, allyle, allyléther, vinyléther, époxy ou styryle ; et
n = 1 à 4,
les substituants éventuellement présents sur les radicaux Y et R étant des atomes d'halogène ou des groupes COOH, OH, alcoxy en C₁-C₁₂, -N⁺[alkyl(C₁-C₁₂)]₃, -O-P=O(OH)₂ et/ou -P=O(OH)₂.

2. Monomères hybrides selon la revendication 1, caractérisés en ce que les variables de la formule (I) ont, indépendamment les unes des autres, les significations suivantes :
A-B = C-C ou C=C,
X = O,
Z = CH₂=C(CH₃)-CO-,
V = CH₂-O,
Y = COOH, CN ou CO-NH₂,
R = alkyle en C₁-C₄ substitué ou non substitué,
U = CO-OR¹ ou CO-NHR¹,
R¹ = alkylène en C₁-C₃,
W = O, NH, CO-O ou est absent ;
P = groupe vinyléther, époxy, allyle, styryle ou (méth)acryle ; et/ou
n = 1 ou 2.

3. Monomères hybrides selon la revendication 1 ou 2, caractérisés en ce qu'ils contiennent au moins trois groupes polymérisables selon des mécanismes différents.

4. Procédé pour la préparation des monomères hybrides polymérisables selon l'une des revendications 1 à 3, caractérisé en ce que l'on prépare un composé norbornène ou norbornadiène de formule (IV), par une réaction de Diels-Alder d'un composé diène (méth)acrylique substitué de formule (II) avec un composé diénophile substitué de formule (III), et on fait réagir le composé (IV), dans le cadre d'une substitution nucléophile, avec le produit de départ contenant des groupes polymérisables P-W-H 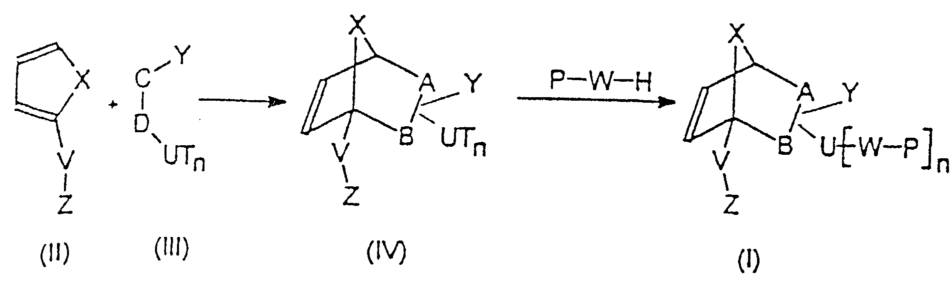 formules dans lesquelles
C-D = C=C ou C≡C ;
H représente un atome d'hydrogène et
T représente un atome d'halogène, OH ou OR,
et les autres variables sont telles que définies dans la revendication 1.

5. Utilisation des monomères hybrides polymérisables selon l'une des revendications 1 à 3, en tant que matériau dentaire ou composant de matériau dentaire.

6. Utilisation selon la revendication 5, dans laquelle le matériau dentaire est un adhésif pour dentine.

7. Matériau dentaire ayant une teneur en monomère hybride polymérisé et/ou non polymérisé, selon l'une des revendications 1 à 3.

8. Polymères ou copolymères, caractérisés en ce qu'ils peuvent être obtenus par polymérisation ou copolymérisation des monomères hybrides selon l'une des revendications 1 à 3.
